# EUROPEAN PATENT SPECIFICATION

(11) **EP 0 973 940 B1**
(45) Date of publication and mention of the grant of the patent: **02.07.2008**
(21) Application number: 98907911.6
(22) Date of filing: 18.03.1998
(51) Int. Cl.: C12Q 1/68, C12N 15/10, C12N 9/00

(54) **AN IN VITRO METHOD FOR THE CONSTRUCTION OF A DNA LIBRARY**
EIN IN-VITRO-VERFAHREN ZUR HERSTELLUNG EINER DNA-BIBLIOTHEK
METHODE DE PRODUCTION IN VITRO D'UNE BIBLIOTHEQUE D'ADN

(30) Priority: 18.03.1997 DK 30797; 17.04.1997 DK 43497; 30.05.1997 DK 62597
(43) Date of publication of application: 26.01.2000
(73) Proprietor: Novozymes A/S, 2880 Bagsvaerd (DK)
(72) Inventor: VIND, Jesper, DK-2880 Bagsværd (DK)
(86) International application number: PCT/DK1998/000104
(87) International publication number: WO 1998/041653

(56) References cited:
- WO-A-98/01581
- US-A- 5 629 179

## Description

### FIELD OF THE INVENTION

The present invention relates to optimizing DNA sequences in order to (a) improve the properties of a protein of interest by artificial generation of genetic diversity of a gene encoding the protein of interest by the use of the so-called gene- or DNA shuffling technique to create a large library of "genes", expressing said library of genes in a suitable expression system and screening the expressed proteins in respect of specific characteristics to determine such proteins exhibiting desired properties or (b) improve the properties of regulatory elements such as promoters or terminators by generation of a library of these elements, transforming suitable hosts therewith in operable conjunction with a structural gene, expressing said structural gene and screening for desirable properties in the regulatory element.

### BACKGROUND OF THE INVENTION

It is generally found that a protein performing a certain bioactivity exhibits a certain variation between genera and even between members of the same species differences may exist. This variation is of course even more outspoken at the genomic level.

This natural genetic diversity among genes coding for proteins having basically the same bioactivity has been generated in Nature over billions of years and reflects a natural optimization of the proteins coded for in respect of the environment of the organism in question.

In today's society the conditions of life are vastly removed from the natural environment and it has been found that the naturally occurring bioactive molecules are not optimized for the various uses to which they are put by mankind, especially when they are used for industrial purposes.

It has therefore been of interest to industry to identify such bioactive proteins that exhibit optimal properties in respect of the use to which it is intended.

This has for many years been done by screening of natural sources, or by use of mutagenesis. For instance, within the technical field of enzymes for use in e.g. detergents, the washing and/or dishwashing performance of e.g. naturally occurring proteases, lipases, amylases and cellulases have been improved significantly, by *in vitro* modifications of the enzymes.

In most cases these improvements have been obtained by site-directed mutagenesis resulting in substitution, deletion or insertion of specific amino acid residues which have been chosen either on the basis of their type or on the basis of their location in the secondary or tertiary structure of the mature enzyme (see for instance US patent no. 4,518,584).

In this manner the preparation of novel polypeptide variants and mutants, such as novel modified enzymes with altered characteristics, e.g. specific activity, substrate specificity, thermal, pH and salt stability, pH-optimum, pI, Kₘ, Vₘₐₓ etc., has successfully been performed to obtain polypeptides with improved properties.

For instance, within the technical field of enzymes the washing and/or dishwashing performance of e.g. proteases, lipases, amylases and cellulases have been improved significantly.

An alternative general approach for modifying proteins and enzymes has been based on random mutagenesis, for instance, as disclosed in US 4,894,331 and WO 93/01285

As it is a cumbersome and time consuming process to obtain polypeptide variants or mutants with improved functional properties a few alternative methods for rapid preparation of modified polypeptides have been suggested.

Weber et al., (1983), Nucleic Acids Research, vol. 11, 5661, describes a method for modifying genes by *in vivo* recombination between two homologous genes. A linear DNA sequence comprising a plasmid vector flanked by a DNA sequence encoding alpha-1 human interferon in the 5'-end and a DNA sequence encoding alpha-2 human interferon in the 3'-end is constructed and transfected into a rec A positive strain of E. *coli.* Recombinants were identified and isolated using a resistance marker.

Pompon et al., (1989), Gene 83, p. 15-24, describes a method for shuffling gene domains of mammalian cytochrome P-450 by *in vivo* recombination of partially homologous sequences in *Saccharomyces cerevisiae* by transforming *Saccharomyces cerevisiae* with a linearized plasmid with filled-in ends, and a DNA fragment being partially homologous to the ends of said plasmid.

In WO 97/07205 a method is described whereby polypeptide variants are prepared by shuffling different nucleotide sequences of homologous DNA sequences by *in vivo* recombination using plasmidic DNA as template.

US patent no. 5,093,257 (Assignee: Genencor Int. Inc.) discloses a method for producing hybrid polypeptides by *in vivo* recombination. Hybrid DNA sequences are produced by forming a circular vector comprising a replication sequence, a first DNA sequence encoding the amino-terminal portion of the hybrid polypeptide, a second DNA sequence encoding the carboxy-terminal portion of said hybrid polypeptide. The circular vector is transformed into a rec positive microorganism in which the circular vector is amplified. This results in recombination of said circular vector mediated by the naturally occurring recombination mechanism of the rec positive microorganism, which include prokaryotes such as *Bacillus* and *E. coli,* and eukaryotes such as *Saccharomyces cerevisiae.*

One method for the shuffling of homologous DNA sequences has been described by Stemmer (Stemmer, (1994), Proc. Natl. Acad. Sci. USA, Vol. 91, 10747-10751; Stemmer, (1994), Nature, vol. 370, 389- 391). The method concerns shuffling homologous DNA sequences by using *in vitro* PCR techniques. Positive recombinant genes containing shuffled DNA sequences are selected from a DNA library based on the improved function of the expressed proteins.

The above method is also described in WO 95/22625. WO 95/22625 relates to a method for shuffling of homologous DNA sequences. An important step in the method described in WO 95/22625 is to cleave the homologous template double-stranded polynucleotide into random fragments of a desired size followed by homologously reassembling of the fragments into full-length genes.

A disadvantage inherent to the method of WO 95/22625 is, however, that the diversity generated through that method is limited due to the use of homologous gene sequences (as defined in WO 95/22625).

Another disadvantage in the method of WO 95/22625 lies in the production of the random fragments by the cleavage of the template double-stranded polynucleotide.

A further reference of interest is WO 95/17413 describing a method of gene or DNA shuffling by recombination of specific DNA sequences - so-called design elements (DE) - either by recombination of synthesized double-stranded fragments or recombination of PCR generated sequences to produce so-called functional elements (FE) comprising at least two of the design elements. According to the method described in WO 95/17413 the recombination has to be performed among design elements that have DNA sequences with sufficient sequence homology to enable hybridization of the different sequences to be recombined.

WO 95/17413 therefore also entails the disadvantage that the diversity generated is relatively limited. Furthermore the method described is time consuming, expensive, and not suited for automatisation.

Despite the existence of the above methods there is still a need for better iterative *in vitro* recombination methods for preparing novel polypeptide variants. Such methods should also be capable of being performed in small volumes, and amenable to automatisation.

### SUMMARY OF THE INVENTION

The present invention concerns briefly the utilization of template shift of a newly synthesized DNA strand during *in vitro* DNA synthesis in order to achieve DNA shuffling. By using this technique it is possible to obtain such results in a more expedient manner, and to some extent even a greater variation than in the above mentioned methods.

The method of the invention is also very well suited for adaption to automatisation.

In a preferred embodiment the technique is used in combination with an error-prone polymerase thereby introducing an even greater variation in the library created.

More specifically the present invention relates to a method for the construction of a library of recombined homologous polynucleotides from a number of different starting single or double stranded parental DNA templates and primers by induced template shifts during an *in vitro* polynucleotide synthesis using a polymerase, wherein
A. extended primers or polynucleotides are synthesized by
   a) denaturing parental double stranded DNA templates to produce single stranded templates,
   b) annealing said primers to the single stranded DNA templates,
   c) extending said primers by initiating synthesis by use of said polymerase,
   d) cause arrest of the synthesis by raising the temperature or by adding DMSO, and
   e) denaturing the double strand to separate the extended primers from the templates,
B. a template shift is induced by
   a) isolating the newly synthesized single stranded extended primers from the templates and repeating steps A.b) to A.e) using said extended primers produced in (A) as both primers and templates, or
   b) repeating steps A.b) to A.e),
C. the above process is terminated after an appropriate number of cycles of process steps A. and B.a), A. and B.b), or combinations thereof, and
D. optionally the produced polynucleotides are amplified in a standard PCR reaction with specific primers to selectively amplify homologous polynucleotides of interest.

In specific embodiments various modifications can be made in the process of the invention. For example it is advantageous to apply a defective polymerase either an error-prone polymerase to introduce mutations in comparison to the templates, or a polymerase that will discontinue the polynucleotide synthesis prematurely to effect the arrest of the reaction.

Further modifications will be described below.

### DEFINITIONS

Prior to discussing this invention in further detail, the following terms will first be defined.

"Shuffling": The term "shuffling" means recombination of nucleotide sequence fragment(s) between two or more homologous polynucleotides resulting in output polynucleotides (i.e. polynucleotides having been subjected to a shuffling cycle) having a number of nucleotide fragments exchanged, in comparison to the input polynucleotides (i.e. starting point homologous polynucleotides).

"Homology of DNA sequences or polynucleotides" In the present context the degree of DNA sequence homology is determined as the degree of identity between two sequences indicating a derivation of the first sequence from the second. The homology may suitably be determined by means of computer programs known in the art, such as GAP provided in the GCG program package (Program Manual for the Wisconsin Package, Version 8, August 1994, Genetics Computer Group, 575 Science Drive, Madison, Wisconsin, USA 53711)(Needleman, S.B. and Wunsch, C.D., (1970), Journal of Molecular Biology, 48, 443-453).

"Homologous": The term "homologous" means that one single-stranded nucleic acid sequence may hybridize to a complementary single-stranded nucleic acid sequence. The degree of hybridization may depend on a number of factors including the amount of identity between the sequences and the hybridization conditions such as temperature and salt concentration as discussed later (*vide infra*).

Using the computer program GAP (*vide supra)* with the following settings for DNA sequence comparison: GAP creation penalty of 5.0 and GAP extension penalty of 0.3, it is in the present context believed that two DNA sequences will be able to hybridize (using low stringency hybridization conditions as defined below) if they mutually exhibit a degree of identity preferably of at least 70%, more preferably at least 80%, and even more preferably at least 85%.

"heterologous": If two or more DNA sequences mutually exhibit a degree of identity which is less than above specified, they are in the present context said to be "heterologous".

"Hybridization:" Suitable experimental conditions for determining if two or more DNA sequences of interest do hybridize or not is herein defined as hybridization at low stringency as described in detail below.

Molecules to which the oligonucleotide probe hybridizes under these conditions are detected using a x-ray film or a phosphoimager.

"primer": The term "primer" used herein especially in connection with a PCR reaction is an oligonucleotide (especially a "PCR-primer") defined and constructed according to general standard specifications known in the art ("PCR A practical approach" IRL Press, (1991)).

"A primer directed to a sequence:" The term "a primer directed to a sequence" means that the primer (preferably to be used in a PCR reaction) is constructed to exhibit at least 80% degree of sequence identity to the sequence fragment of interest, more preferably at least 90% degree of sequence identity to the sequence fragment of interest, which said primer consequently is "directed to". The primer is designed to specifically anneal at the sequence fragment or region it is directed towards at a given temperature. Especially identity at the 3' end of the primer is essential.

"Flanking" The term "flanking" used herein in connection with DNA sequences comprised in a PCR-fragment means the outermost partial sequences of the PCR-fragment, both in the 5' and 3' ends of the PCR fragment.

"Polypeptide" Polymers of amino acids sometimes referred to as proteins. The sequence of amino acids determines the folded conformation that the polypeptide assumes, and this in turn determines biological properties and activity. Some polypeptides consist of a single polypeptide chain (monomeric), whereas other comprise several associated polypeptides (multimeric). All enzymes and antibodies are polypeptides.

"Enzyme" A protein capable of catalysing chemical reactions. Specific types of enzymes to be mentioned are such as amylases, proteases, carbohydrases, lipases, cellulases, oxidoreductases, esterases, etc. Of specific interest in relation to the present invention are enzymes used in detergents, such as proteases, lipases, cellulases, amylases, etc.

### DETAILED DESCRIPTION OF THE INVENTION

The present invention relates in its first aspect to a method for the construction of a library of recombined homologous polynucleotides from a number of different starting single or double stranded parental DNA templates and primers by induced template shifts during an *in vitro* polynucleotide synthesis using a polymerase, wherein
A. extended primers or polynucleotides are synthesized by
   a) denaturing a parental double stranded DNA template to produce single stranded templates,
   b) annealing said primers to the single stranded DNA templates,
   c) extending said primers by initiating synthesis by use of said polymerase,
   d) cause arrest of the synthesis by raising the temperature or by adding DMSO, and
   e) denaturing the double strand to separate the extended primers from the templates,
B. a template shift is induced by
   a) isolating the newly synthesized single stranded extended primers from the templates and repeating steps A.b) to A.e) using said extended primers produced in (A) as both primers and templates, or
   b) repeating steps A.b) to A.e),
C. the above process is terminated after an appropriate number of cycles of process steps A. and B.a), A. and B.b), or combinations thereof, and
D. optionally the produced polynucleotides are amplified in a standard PCR reaction with specific primers to selectively amplify homologous polynucleotides of interest.

According to the invention the polymerase may be a DNA or a RNA polymerase, specific polymerases to be mentioned are such as DNA polymerases like T4 polymerase, T7 polymerase , E. coli DNA polymerase I or the Klenow fragment of DNA polymerase I, or thermostable polymerases such as Taq, Amplitaq, Vent, Pwo.

One of the advantages of the invention is that it makes it possible to control the length of the extension of the primers in the reaction in a convenient manner.

This can be accomplished by various means such as choice of polymerase, the physical and chemical conditions during the action of the polymerase, e.g. pH, temperature, buffer, salt concentration, and addition of various chemicals.

It is known that various polymerases carry out the DNA synthesis at different rates (nucleotides incorporated pr. second). For example has the Klenow fragment of DNA polymerase I a limited extension rate compared to e.g. the Taq polymerase (Sambrook et al. 1989).

Polymerases also display differences in processivity, which is the average number of nucleotides incorporated before the polymerase dissociates from the template/extended primer; again the Klenow polymerase is an example of a polymerase with limited processivity.

The choice of polymerase is therefore an important means in controlling the average extension of the primers.

These conditions may also exert an influence on the fidelity of the polymerase (the rate by which point mutations are introduced; HIV reverse transcriptase is an example of a polymerase of low fidelity), a parameter useful in combining shuffling and mutagenesis.

In specific embodiments various modifications can be made in the process of the invention. For example it is advantageous to apply a defective polymerase either an error-prone polymerase to introduce mutations in comparison to the templates, or a polymerase that will discontinue the polynucleotide synthesis prematurely to effect the arrest of the reaction. Such a defective polymerases that could be mentioned is a Klenow polymerase having low processivity.

In another embodiment of the invention polymerase will be added after each cycle, if the polymerase used is not thermostable.

According to the invention the starting single or double stranded parental templates may be different in that they contain different point mutations in the same native polynucleotide (gene), or they can be homologous polynucleotides (genes) isolated from nature, which may be amplified by PCR, or they can be combinations thereof. The templates used in the process of the invention are hereby homologous showing an identity at the DNA level of e.g. more than 95%, 90%, 85%, 80%, 75%, 70%, 65%, 60%, 55%, or even more than 50% identity.

It may be advantageous to use pre-selected templates comprising mutations with improved properties of interest. The present recombination method of the invention will then recombine said improved mutations for subsequent screening for even further improvements in the properties of interest.

Said pre-selected templates with improved properties of interest may have been identified by standard procedures in the art comprising e.g. i) error-prone PCR of templates of interest followed by ii) screening/selection for templates with improved characteristic of interest. The mutagenesis frequency (low or high mutagenesis frequency) of the error-prone PCR step is preferably adjusted in relation to the subsequent screening capacity, i.e. if the screening capacity is limited the error-prone PCR frequency is preferably low (i.e. one to two mutations in each template) (see WO 92/18645 for further details).

The arrest of the polymerase reaction in step A.d) may as indicated above be obtained in different ways, such as by raising the temperature, or adding specific reagents as described in WO 95/17413.

When raising the temperature for this purpose, it is preferred to use temperatures between 90°C and 99°C.

When using chemical agents DMSO is a possibility. Appropriate procedures are mentioned in e.g. WO 95/17413.

The process of the invention uses annealing of the primers to the templates in step 1.b. In this context the annealing may be random or specific, meaning either anywhere on the polynucleotide or at a specific position depending on the nature of the primer.

Also, the primers to be used may be completely random primers (NNNNNNNNNNN) (N meaning a mixture of the four bases (A, T, G, C) is used at a particular position in the primer during synthesis), semi-random primers, or specific primers.

If the extended primers produced are to be separated from the primers during the process it is convenient to use labeled templates in order to provide a simple means for separation, a preferred marker is biotin or digoxigenin.

According to the invention the number of cycles necessary will be less than 500, in most cases less than 200, and normally less than 100 cycles.

In an embodiment of the invention the above *in vitro* shuffling is combined with a subsequent *in vivo* shuffling by methods such as those described in WO 97/07205.

In an embodiment it is contemplated that the polypeptides of interest encoded by the shuffled library are expressed as a suitable fusion protein (e.g. as a hybrid with gIII of bacteriphage M13/fd) in order to display said recombined polypeptide on the surface of phage or bacteria.

The use of partial random (semi-random) or completely random primers (mixtures of bases in a selected number or all positions in the primer) as initiation point for the DNA synthesis provide certain novel possibilities for the combined use of shuffling and random mutagenesis.

It is often associated with difficulties to obtain an *in vitro* recombination of polynucleotides that display relatively limited homology. By the use of an embodiment of the invention even very diverse polynucleotides can be forced to recombine.

According to that embodiment at least two templates (or two pools of diverse templates) are applied. The novel synthesis of the one polynucleotide can then be based on only one strand (i.e. either the sense or the anti-sense strand), and the synthesis of the other polynucleotide is based the opposite strand.

This can be accomplished by isolating the complementary strands from the two templates, e.g. by having these strands labeled by biotin. Synthesis of DNA is initiated by annealing either specific, partly random or completely random primers to these templates and adding a suitable polymerase. This can be performed as either separate reactions for the different templates or in just one reaction. Synthesis should preferably be performed under conditions that favor production of relatively short new fragments. These fragments can subsequently be isolated from the templates based on the affinity label. A PCR reaction is carried out on these fragments and as the starting material originates from two different strands, the newly synthesized fragments must recombine in order to produce full length PCR products - a kind of forced recombination.

Also for this embodiment rapid PCR with short or no extension time can be applied advantageously in order to enhance recombination, especially if pools of templates are used for the two strands.

The length of the primer and the annealing temperature utilized in the process determines if random primers will anneal and the number of mismatches between the template and the primer that can be accommodated. By varying the primer length and the annealing temperature the method of the invention provides a means for achieving random mutagenesis within a certain nucleotide window representing the length of the primer. The method of the invention thereby provides substantial benefits compared to other random mutagenesis approaches, especially the high probability for several base substitutions close to each other in the primary sequence, e.g. the use of a completely random 20'mer (mixture of all four nucleotides in all 20 positions) will according to theory under given experimental conditions give a certain reasonably high probability for having several base substitutions close to each other.

Error prone PCR (=high mutagenesis frequency PCR) does not provide this possibility. Error prone PCR provides a very low probability for having more than one base substitution within one codon (coding for one amino acid in a translated polypeptide).

Obviously the substitution of only one base within a codon doesn't provide total random mutagenesis (at protein level) as only a limited set of amino acid substitutions can be obtained by one base substitution at DNA level (e.g. Methionine encoded by ATG-codon requires three base substitution to become the TGT or TGC-codon encoding Cysteine).

In one embodiment of the invention the process is therefore performed by using random or semi-random primers having a length of from 6 to 200 bp, preferably from 10 to 70 bp, and better from 15 to 40 bp.

One of the advantages in the method of the invention is the robustness. In some embodiments the constant presence of full length template provides a further advantage avoiding PCR contamination problems. Furthermore it is much less laborius, with less hands on, than other described methods, thereby providing excellent possibilities for automation.

### PCR-primers:

The PCR primers are constructed according to the standard descriptions in the art. Normally they are 10-75 base-pairs (bp) long. However, for the specific embodiment using random or semi-random primers the length may be substantially longer as indicated above.

### PCR-reactions:

If not otherwise mentioned the PCR-reaction performed according to the invention are performed according to standard protocols known in the art.

The term "Isolation of PCR fragment" is intended to cover as broad as simply an aliquot containing the PCR fragment. However preferably the PCR fragment is isolated to an extend which remove surplus of primers, nucleotides templates etc..

In an embodiment of the invention the DNA fragment(s) is(are) prepared under conditions resulting in a low, medium or high random mutagenesis frequency.

To obtain low mutagenesis frequency the DNA sequence(s) (comprising the DNA fragment(s)) may be prepared by a standard PCR amplification method (US 4,683,202 or Saiki et al., (1988), Science 239, 487 - 491).

A medium or high mutagenesis frequency may be obtained by performing the PCR amplification under conditions which increase the misincorporation of nucleotides, for instance as described by Deshler, (1992), GATA 9(4), 103-106; Leung et al., (1989), Technique, Vol. 1, No. 1, 11-15.

It is also contemplated according to the invention to combine the PCR amplification (i.e. according to this embodiment also DNA fragment mutation) with a mutagenesis step using a suitable physical or chemical mutagenizing agent, e.g., one which induces transitions, transversions, inversions, scrambling, deletions, and/or insertions.

### Screening and selection

In the context of the present invention the term "positive polypeptide variants" means resulting polypeptide variants possessing functional properties which has been improved in comparison to the polypeptides producible from the corresponding input DNA sequences. Examples, of such improved properties can be as different as e.g. enhance or lowered biological activity, increased wash performance, thermostability, oxidation stability, substrate specificity, antibiotic resistance etc.

Consequently, the screening method to be used for identifying positive variants depend on which property of the polypeptide in question it is desired to change, and in what direction the change is desired.

A number of suitable screening or selection systems to screen or select for a desired biological activity are described in the art. Examples are:
Strauberg et al. (Biotechnology 13: 669-673 (1995) describes a screening system for subtilisin variants having Calcium-independent stability;
Bryan et al. (Proteins 1:326-334 (1986)) describes a screening assay for proteases having a enhanced thermal stability; and
PCT-DK96/00322 describes a screening assay for lipases having improved wash performance in washing detergents.

An embodiment of the invention comprises screening or selection of recombinant protein(s), wherein the desired biological activity is performance in dish-wash or laundry detergents. Examples of suitable dish-wash or laundry detergents are disclosed in PCT-DK96/00322 and WO 95/30011.

If, for instance, the polypeptide in question is an enzyme and the desired improved functional property is the wash performance, the screening may conveniently be performed by use of a filter assay based on the following principle:

The recombination host cell is incubated on a suitable medium and under suitable conditions for the enzyme to be secreted, the medium being provided with a double filter comprising a first protein-binding filter and on top of that a second filter exhibiting a low protein binding capability. The recombination host cell is located on the second filter. Subsequent to the incubation, the first filter comprising the enzyme secreted from the recombination host cell is separated from the second filter comprising said cells. The first filter is subjected to screening for the desired enzymatic activity and the corresponding microbial colonies present on the second filter are identified.

The filter used for binding the enzymatic activity may be any protein binding filter e.g. nylon or nitrocellulose. The top-filter carrying the colonies of the expression organism may be any filter that has no or low affinity for binding proteins e.g. cellulose acetate or Durapore^{®}. The filter may be pre-treated with any of the conditions to be used for screening or may be treated during the detection of enzymatic activity.

The enzymatic activity may be detected by a dye, fluorescence, precipitation, pH indicator, IR-absorbance or any other known technique for detection of enzymatic activity.

The detecting compound may be immobilized by any immobilizing agent e.g. agarose, agar, gelatin, polyacrylamide, starch, filter paper, cloth; or any combination of immobilizing agents.

If the improved functional property of the polypeptide is not sufficiently good after one cycle of shuffling, the polypeptide may be subjected to another cycle.

In an embodiment of the invention wherein homologous polynucleotides representing a number of mutations of the same gene is used as templates at least one shuffling cycle is a backcrossing cycle with the initially used DNA fragment, which may be the wild-type DNA fragment. This eliminates non-essential mutations. Non-essential mutations may also be eliminated by using wild-type DNA fragments as the initially used input DNA material.

Also contemplated to be within the invention is polypeptides having biological activity such as insulin, ACTH, glucagon, somatostatin, somatotropin, thymosin, parathyroid hormone, pituary hormones, somatomedin, erythropoietin, luteinizing hormone, chorionic gonadotropin, hypothalamic releasing factors, antidiuretic hormones, thyroid stimulating hormone, relaxin, interferon, thrombopoeitin (TPO) and prolactin.

A requirement to the starting parental DNA sequences, encoding the polypeptide(s), to be shuffled, is that they are at least 50%, 60%, 70%, 80%, 90%, or 95% homologous. DNA sequences being less homologous will have less inclination to interact and recombine.

It is also contemplated according to the invention to shuffle parental polynucleotides that are homologous as indicated above originating from wild type organisms of different genera.

Further, the starting parental templates to be shuffled may preferably have a length of from about 50 bp to 20 kb, preferably about 100 bp to 10 kb, more preferred about 200 bp to 7 kb, especially about 400 bp to 2 kb.

The starting parental DNA sequences may be any DNA sequences including wild-type DNA sequences, DNA sequences encoding variants or mutants, or modifications thereof, such as extended or elongated DNA sequences, and may also be the outcome of DNA sequences having been subjected to one or more cycles of shuffling (i.e. output DNA sequences) according to the method of the invention or any other method (e.g. any of the methods described in the prior art section).

When using the method of the invention the resulting recombined homologous polynucleotides (i.e. shuffled DNA sequences), have had a number of nucleotide fragments exchanged. This results in replacement of at least one amino acid within the polypeptide variant, if comparing it with the parent polypeptide. It is to be understood that also silent exchanges are contemplated (i.e. nucleotide exchange which does not result in changes in the amino acid sequence).

### MATERIALS AND METHODS

Specific Method Used in the Examples:
A) DNA encoding different enzyme variants of the same gene or different enzymes having the same type of activity encoded by homologous genes are mixed. The DNA is provided as either PCR fragments, plasmid, phage or genomic DNA.
B) The resulting pool of DNA is mixed with DNA Polymerase, dNTP, a suitable buffer and primers (being either random oligomers (length of 6-30 nucleotides) or specific oligomers (length of 6-50 nucleotides) or a combination of both types).
C) The PCR mixture is put into a PCR thermocycler (either cold or hot) in a suitable tube.
   c1) The thermocycler is heated to a temperature of 90-100°C for a period of time(typically 1-10 min) in order to denature the DNA templates.
   c2) Thereafter the following procedure (cycle) is followed (repeated): The template is denatured (typically 90-100°C for 0-5 minutes). Then the temperature is lowered (typically to a value between 10°C and 90°C for 0-5 minutes) to allow annealing of the primer to the single stranded template. Now the temperature is raised again to denaturation temperature (90-100°C) allowing small extension of the primer to be synthesized by the DNA polymerase during ramping. Alternatively a short extension period (typically 0-30 seconds at 70-75°C) can be introduced to allow larger extensions of the primers to be generated. When the temperature reaches a value where denaturation takes place, the extended primers and templates are again separated. This procedure can be repeated (typically between 1 to 99 cycles).
D) Having performed the desired number of cycles the generated small DNA polymers can be purified from the oligomers used as primers. One way is to isolate and clone a specific amplified band containing the gene coding for the polypeptide of interest into a suitable vector. This can be done either on agarose gel (typically isolating fragments between 50 to 1000 base pairs), by beads (using an affinity label on either templates or primers) or through columns.
E) Then the purified (or the not purified) DNA polymers can be assembled in a standard PCR reaction (for instance 94°C, 5 minutes, (94°C, 30 sec; 55°C, 30 sec; 72°C, 2 min)* 25, 72°C 5 minutes, 4°C).

Specific primers or DNA polymers generated by specific primers can be added in order to generate a specific DNA polymer containing the gene of interest. This As mentioned in point D, this DNA polymer can be purified and cloned into a vector of interest.

### EXAMPLES

### EXAMPLE 1

### Method 1

The strong advantage of the method exemplified here is the robustness and lack of PCR contamination problems, due to the constant presence of parental template. Furthermore this method is less labor demanding than methods described in the prior art, thereby providing excellent possibilities for automation.

Nine different plasmids containing DNA sequences encoding 9 different variants of the *H. lanuginosa* lipase gene, were mixed in equimolar amounts. The variant genes contained from two to seven mutations scattered throughout the gene.

The DNA sequence of the *H. lanuginosa* lipase gene and the amino acid sequence of the lipase are disclosed in EP 0 305 216

The variants are indicated according to standard terminology as described in e.g. EP 0 396 608, and WO 97/07202.

The following 9 variant genes were shuffled:
1'. N94K+D96L+E99K
2'. SPPRRP+N94K+D96L+T231R+N233R+D234R+Q249R
3'. SPPRRP+Al9C+C36A+N94K+D96L+Q249R
4'. STPRRP+N94R
5'. SCIRR+N94K+D96L+E239C+Q249R
6'. D137G+D167G+E210V
7'. D96L+E99K+V187A
8'. SPPRRP+D57G+N94K+D96L+Q249R
9'. N94R+F95L

The following components where mixed in a microtube:
2 µl plasmid mixture (0.15 µg/µl), specific primers flanking the gene (1 pmol/µl), 2 µl 2.5 mM dNTP, 2.5 mM MgCl₂, 2 µl 10* taq buffer (Perkin Elmer), 0.5 µl taq enzyme in a total volume of 20 µl.
The tube was set in a Perkin Elmer 2400 thermocycler. The following PCR-program was run:(94°C, 5 minutes) 1 cycle: (94°C , 30 seconds, 70°C, 0 seconds) 99 cycles (72°C, 2 minutes, 4°C indefinite) 1 cycle
The PCR-reaction was run on a 1.5 % agarose gel. A DNA-band of the specific expected size was cut out of the agarose gel and purified using JETsorb (from GENOMED Inc.). The purified PCR-product was cloned into a TA-vector (from Invitrogen (the original TA cloning kit). The ligated product was transformed into a standard *Escherichia coli* strain (DH5a).
20 transformants where fully sequenced across the gene of interest.

### Result:

The following 20 variants were found:
1. D137G+D167G+E210V+Y213C
2. SPPRRP+D57G+N94K+D96L+Q249R
3. N94R+F95L
4. SPPRRP+D137G+D167G+E210V
5. N94K+D96L+E99K+V187A+T2671
6. D137G+D167G+E210V
7. N94K+D96L+E99K+V187A
8. D57G+N94R+F95L+Q249R
9. N94K+D96L+E99K+E210V
10. SPPRRP+A19C+C36A+N94K+D96L
11. N94R+F95L
12. D137G+D167G+E210V
13. N94K+D96L+Q249R
14. SPPRRP+Q15P+A19C+C36A+N94K+D96L
15. SPPRRP+N94K+D96L+T231R+N233R+D234R+Q249R
16. D137G+D167G+E210V
17. SCIRR+N94K+D96L+Q249R
18. N94K+D96L+E99K
19. N94R+F95L
20. SPPRRP+N94R+F95L+F113S+Q249R

Nearly all mutations where represented (19 of 20) indicating little bias for specific templates.

| Statistics: | |
|---|---|
| Not shuffled | 10 |
| Shuffled between at least 2 templates | 8 |
| Shuffled between at least 3 templates | 2 |

The shuffled sequences can then be subcloned from the *E.coli* TA vector into the yeast vector pJSO26 as a BamHI-XbaI fragment (see WO 97/07205), and e.g. screened for new shuffled sequences with improved performance in detergents (see WO 97/07205).

### EXAMPLE 2

### Method 2:

PCR products of 10 different lipase variant genes were generated as above and pooled in equimolar amounts.

The following 10 mutant genes were shuffled.
1'. D137G+D167G+E210V
2'. D96L+E99K+V187A
3'. N94K+D96L+E99K
4'. SPPRRP+D57G+N94K+D96L+Q249R
5'. D111N+F211A+G225P
6'. SPPRRP+N94K+D96L+T231R+N233R+D234R+Q249R
7'. SPPRRP+A19C+C36A+N94K+D96L+Q249R
8'. STPRRP+N94R
9'. N94R+F95L
10'. SCIRR+N94K+D96L+E239C+Q249R

The following mixture was generated in a suitable tube:
1 µl PCR mixture (0.1 µg), decamer random primer (300 pmol), 2 µl 10* Klenow buffer (Promega), 0.25 mM dNTP, 2.5 mM MgCl₂ in a total volume of 20 µl.

The mixture was set in a PE2400 thermocycler where the following program was run: 96°C, 5 minutes, 25°C 5 minutes, 0.5 ml Klenow enzyme was added, 25°C 60 minutes, 35°C 90 minutes.

This procedure generated a high number of small DNA polymers originating from all parts of the gene
10 µl was taken out for test on agarose gel.
10 µl PCR mixture (0.25 mM dNTP, 1 µl 10* Taq buffer (Perkin Elmer), 2.5 mM MgCl₂, 0.5 µl Taq enzyme) was added to the 10 µl in the tube in the thermocycler. Then the following standard PCR-program was run: (94°C, 5 minutes) 1 cycle, (94°C 30 seconds, 45°C, 30 seconds, 72°C 30 seconds) 25 cycles, 72°C 7 minutes, 4°C indefinite.

The PCR products were run on a 1.5% agarose gel. A clear unbiased smear was seen. DNA between 400 and 800 bp was isolated from the gel.

Half of the purified PCR product was mixed in a tube with two specific primers (40 pmol) flanking the gene of interest, 0.25 mM dNTP, 2 µl 10* Taq buffer, 2.5 mM MgCl₂. Then the following standard PCR-program was run: (94°C , 5 minutes) 1 cycle, (94°C 30 seconds, 50°C, 30 seconds, 72°C 30 seconds) 25 cycles, 72°C 7 minutes, 4°C indefinite.

The PCR product was run on a 1.5% agarose gel. A specific though weak band of the expected size was isolated. Additional PCR was run using specific primers (as mentioned above) in order to amplify the PCR-product before cloning.

The PCR-product and the desired vector were cut with the appropriate restriction enzymes (BamHI/XhoI). The vector and the PCR product were run on a 1.5% agarose gel, and purified from the gel.

The cut PCR-product and the cut vector were mixed in a ligase buffer with T4 DNA ligase (Promega). After overnight ligation at 16°C the mixture was transformed into E. *coli* strain DH5a.

19 clones were fully sequenced across the gene.

### Result:

The following 19 variants were found:
1. STPRRP+N94R+N233R+D234R+Q249R
2. SPPRRP+N233R+D234R+Q249R
3. D96L+D167G+Q249R
4. N94R
5. D167G
6. SPPRRP+A19C+A28T+N94K+D96L+D111N+E239C
7. SPPRRP+A19C+C36A+N94K+D96L+Q249R
8. N94K+D96L
9. N25T+D57G+N94R+E99K+D167G+T231R+N233R+D234R+Q249R
10. N94R+Q249R
11. D167G
12. D167G
13. T32I+N94R+F95L+D167G+Q249R
14. E87K+N94K+D96L
15. N94R+F95L+Q249R
16. N94K+D96L+D111N
17. STPRRP+S17T
18. N94K+D96L+V187A
19. SPPRRP+D57G+N94K+D96L+D111N+L151S

All template variants were represented indicating little bias for specific templates.

There were no apparent hot spots with regard to mutation exchange and it seems to be evenly distributed along the gene

| Statistics: | |
|---|---|
| Not shuffled | 1 |
| Shuffled between at least 2 templates | 10 |
| Shuffled between at least 3 templates | 6 |
| Shuffled between at least 4 templates | 1 |
| Shuffled between at least 5 templates | 0 |
| Shuffled between at least 6 templates | 1 |

The shuffled sequences can then be subcloned from the E.coli TA vector into the yeast vector pJSO26 as a BamHI-XbaI fragment (see WO 97/07205), and e.g. screened for new shuffled sequences with improved performance in detergents (see WO 97/07205).

### Example 3:

### Amylase variant shuffling:

In Example 1, it was shown how a number of multiple variants of *H. lanuginosa* lipase were shuffled. In a similar manner, variants of Bacillus α-amylases can be shuffled.

Earlier patent applications have identified variants of various α-amylases from Bacillus species improved for particular properties, *e.g.* thermostability, stability under Calcium-depleted conditions, improved wash-performance etc. (see WO95/10603, WO96/23874, WO96/23873, and PCT/DK97/00197).

Variants of *B. licheniformis* α-amylase amyL can be shuffled as follows. The variants are all located in the *B. subtilis* expression vector pDN1528 described in WO95/10603.

The experiment is carried out under the exact same conditions as Example 1 except that the flanking 27mer primers used to initiate DNA synthesis were different.

The PCR amplified band of approximately 1500 bp is purified from an agarose gel and cloned as described in Example1. Alternatively restriction sites located within the amyL gene can be utilized to clone the library of shuffled genes into either Bacillus plasmid pDN1528 or an E. *coli* vector containing the wild type amyL gene, e.g. pJeEN1 described in WO96/23874

### Example 4:

Shuffling of two genes encoding homologous α-amylases: amyL and the amylase identified by SEQ.ID no2 (amino acid) and SEQ.ID no. 5 (DNA) described in WO96/23873.
The forward strand (identical to the mRNA) of amyL can be amplified in a PCR using standard conditions.

The forward strand is separated from the reverse strand based on its affinity to streptavidin coated magnetic beads and denaturation of the two strands with NaOH.
Similarly the reverse strand (complementary to mRNA) of the amylase encoded by SEQ.ID no5 (WO96/23873) can be amplified and isolated

Two primer strands are used as templates in a PCR: (94°C 5 minutes) + 99 cycles of (94°C, 30 seconds; 60°C, 0 seconds)+ (72°C, 5 minutes) using random primers of various lengths, Taq polymerase and standard buffer conditions as described in Example 1.

The resulting approximately 1500 bp product is cloned either as TA cloning as described in Example one (for verification of the sequence of the resulting clone) or into Bacillus vector, pTVB110 utilizing SfiI and PstI restriction sites.

The original template can be removed from the PCR at any step (e.g. after 5, 10 or 20 cycles) based on the biotin tag).

## Claims

1. A method for the construction of a library of recombined homologous polynucleotides from a number of different starting single or double stranded parental DNA templates and primers by induced template shifts during an *in vitro* polynucleotide synthesis using a polymerase, wherein
A) extended primers or polynucleotides are synthesized by
a) denaturing a parental double stranded DNA template to produce single stranded templates,
b) annealing said primers to the single stranded DNA templates,
c) extending said primers by initiating synthesis by use of said polymerase,
d) cause arrest of the synthesis by raising the temperature or by adding DMSO, and
e) denaturing the double strand to separate the extended primers from the templates;
B) a template shift is induced by
a) isolating the newly synthesized single stranded extended primers from the templates and repeating steps (A)b) to (A)e) using said extended primers produced in (A) as both primers and templates, or
b) repeating steps (A)b) to (A)e);
C) the above process is terminated after an appropriate number of cycles of process steps (A) and (B)a), (A) and (B)b), or combinations thereof; and
D) optionally the produced polynucleotides are amplified in a standard PCR reaction with specific primers to selectively amplify homologous polynucleotides of interest.

2. The method of claim 1, wherein said polymerase is a thermostable DNA polymerase.

3. The method of claim 1, wherein said polymerase is a DNA polymerase, such as T4 polymerase, T7 polymerase , E. coli DNA polymerase I or the Klenow fragment of DNA polymerase I, and said polymerase is added to the reaction mixture after each cycle.

4. The method of claim 1, wherein said polymerase is a RNA polymerase.

5. The method of any of the claims 1 to 4, wherein said polymerase is an error-prone polymerase, such as a reverse transcriptase like HIV reverse transcriptase.

6. The method of any of the claims 1 to 5, wherein said starting single or double stranded parental templates are different in that they contain different point mutations in the same native polynucleotide, or are homologous polynucleotides isolated from nature.

7. The method of claim 6, wherein said starting single or double stranded parental templates exhibit an identity at the DNA level of e.g. more than 95%, 90%, 85%, 80%, 75%, 70%, 65%, 60%, 55%, or even more than 50% identity.

8. The method of any of the claims 1 to 7, wherein the temperature is raised to a value between 90°C and 99°C, preferably 94°C.

9. The method of any of the claims 1 to 8, wherein said primers comprise a population of completely random primers.

10. The method of any of the claims 1 to 8, wherein said primers comprise a population of semi-random primers.

11. The method of any of the claims 1 to 8, wherein said primers comprise a population of specific primers.

12. The method of any of the claims 1 to 11, wherein said primers are labeled, preferably with biotin or digoxigenin.

13. The method of any of the claims 9, 10, or 12, wherein said random or semi-random primers have a length of from 6 to 500 bp, preferably from 15 to 300 bp, and better from 25 to 150 bp.

14. The method of any of the claims 11 or 12, wherein said primers are 6 to 75 bp long.

15. The method of any of the claims 1 to 14, wherein said starting parental DNA templates are at least 50%, 60%, 70%, 80%, 90%, or 95% homologous.

16. The method of claim 15, wherein said starting parental DNA templates originate from wild type organisms of different genera.

17. The method of any of the claims 15 or 16, wherein said starting parental templates have a length of from about 50 bp to 20 kb, preferably about 100 bp to 10 kb, more preferred about 200 bp to 7 kb, especially about 400 bp to 2 kb.

18. The method of any of the claims 1 to 17, wherein said starting parental templates are linear DNA fragments generated by a PCR reaction.

19. The method of any of the claims 1 to 18, wherein said starting parental templates are cloned into suitable vectors, such as a plasmid.

20. The method of any of the claims 1 to 19, wherein said starting parental templates represent a polynucleotide encoding an enzyme, such as a carbonyl hydrolase, carbohydrase, or esterase, such as a protease, lipase, amylase, cellulase, oxidase, or oxido reductase.

21. The method of any of the claims 1 to 19, wherein said starting parental templates represent a polynucleotide encoding a polypeptide having biological activity, such as insulin, ACTH, glucagon, somatostatin, somatotropin, thymosin, parathyroid hormone, pituary hormones, somatomedin, erythropoietin, luteinizing hormone, chorionic gonadotropin, hypothalamic releasing factors, antidiuretic hormones, thyroid stimulating hormone, relaxin, interferon, thrombopoeitin (TPO) and prolactin.

22. The method of any of the claims 1 to 19, wherein said starting parental templates represent a polynucleotide containing a biological function such as transcription initiation (promoter) or termination, translational initiation, operator sites related to expression of genes

23. The method of any of the claims 1 to 22, wherein said number of cycles are less than 500, 200, or less than 100 cycles.

## Patentansprüche

1. Verfahren zum Konstruieren einer Genbank von rekombinierten homologen Polynukleotiden aus einer Anzahl von unterschiedlichen einzel- oder doppelsträngigen Elternausgangs-DNA-Matrizen und Primern durch induzierte Matrizenverschiebungen während einer *in vitro* Polynukleotid-Synthese unter Verwendung einer Polymerase, wobei
A) verlängerte Primer oder Polynukleotide synthetisiert werden durch
a) Denaturieren einer doppelsträngigen Eltern-DNA-Matrize zur Herstellung von einzelsträngigen Matrizen,
b) Anlagern der Primer an die einzelsträngigen DNA-Matrizen,
c) Verlängern der Primer durch Initiieren der Synthese durch Verwenden der Polymerase,
d) Verursachen eines Halts der Synthese durch Anheben der Temperatur oder durch Zugeben von DMSO, und
e) Denaturieren des Doppelstrangs zur Trennung der verlängerten Primer von der Matrize.
B) eine Matrizenverschiebung wird induziert durch
a) Isolieren der neu synthetisierten, einzelsträngigen, verlängerten Primer aus den Matrizen und Wiederholen der Schritte (A)b) bis (A)e) unter Verwendung der in (A) hergestellten verlängerten Primer sowohl als Primer, als auch als Matrizen, oder
b) Wiederholen der Schritte (A)b) bis (A)e);
C) das obige Verfahren wird nach einer angemessenen Anzahl von Zyklen der Verfahrensschritte (A) und (B)a), (A) und (B)b), oder Kombinationen davon, abgeschlossen; und
D) wahlweise werden die hergestellten Polynukleotide in einer Standard-PCR-Reaktion mit spezifischen Primern vervielfältigt, um gezielt homologe Polynukleotide von Interesse zu vervielfältigen.

2. Verfahren nach Anspruch 1, wobei die Polymerase eine thermostabile DNA-Polymerase ist.

3. Verfahren nach Anspruch 1, wobei die Polymerase eine DNA-Polymerase, wie T4-Polymerase, T7-Polymerase, *E. coli* DNA-Polymerase I oder das Klenow-Fragment der DNA-Polymerase I ist, und die Polymerase nach jedem Zyklus zu dem Reaktionsgemisch hinzugefügt wird.

4. Verfahren nach Anspruch 1, wobei die Polymerase eine RNA-Polymerase ist.

5. Verfahren nach einem beliebigen der Ansprüche 1 bis 4, wobei die Polymerase eine fehleranfällige Polymerase ist, wie eine reverse Transkriptase, wie HIV reverse Transkriptase.

6. Verfahren nach einem beliebigen der Ansprüche 1 bis 5, wobei die einzel- oder doppelsträngigen Elternausgangsmatrizen sich unterscheiden, indem sie verschiedene Punktmutationen in dem gleichen nativen Polynukleotid beinhalten, oder aus der Natur isolierte homologe Polynukleotide sind.

7. Verfahren nach Anspruch 6, wobei die einzel- oder doppelsträngigen Elternausgangsmatrizen eine Identität auf der DNA-Ebene von z.B. mehr als 95 %, 90 %, 85 %, 80 %, 75 %, 70 %, 65 %, 60 %, 55 %, oder sogar mehr als 50 % Identität aufweisen.

8. Verfahren nach einem beliebigen der Ansprüche 1 bis 7, wobei die Temperatur auf einen Wert zwischen 90 °C und 99 °C, bevorzugter Weise 94 °C, erhöht wird.

9. Verfahren nach einem beliebigen der Ansprüche 1 bis 8, wobei die Primer einen Bestand von vollständig zufälligen Primern umfasst.

10. Verfahren nach einem beliebigen der Ansprüche 1 bis 8, wobei die Primer einen Bestand von halb-zufälligen Primern umfasst.

11. Verfahren nach einem beliebigen der Ansprüche 1 bis 8, wobei die Primer einen Bestand von spezifischen Primern umfasst.

12. Verfahren nach einem beliebigen der Ansprüche 1 bis 11, wobei die Primer markiert sind, bevorzugter Weise mit Biotin oder Digoxigenin.

13. Verfahren nach einem beliebigen der Ansprüche 9, 10, oder 12, wobei die zufälligen oder halb-zufälligen Primer eine Länge von 6 bis 500 bp, bevorzugter Weise von 15 bis 300 bp und besser von 25 bis 150 bp besitzen.

14. Verfahren nach einem beliebigen der Ansprüche 11 oder 12, wobei die Primer 6 bis 75 bp lang sind.

15. Verfahren nach einem beliebigen der Ansprüche 1 bis 14, wobei die Ausgangseltern-DNA-Matrizen zu mindestens 50 %, 60 %, 70 %, 80 %, 90 %, oder 95 % homolog sind.

16. Verfahren nach Anspruch 15, wobei die Ausgangseltern-DNA-Matrizen aus Wildtyp-Organismen unterschiedlicher Gattungen herstammen.

17. Verfahren nach einem beliebigen der Ansprüche 15 oder 16, wobei die Ausgangselternmatrizen eine Länge von etwa 50 bp bis 20 kb haben, bevorzugter Weise etwa 100 bp bis 10 kb, noch bevorzugter etwa 200 bp bis 7 kb, insbesondere etwa 400 bp bis 2 kb.

18. Verfahren nach einem beliebigen der Ansprüche 1 bis 17, wobei die Ausgangselternmatrizen durch eine PCR-Reaktion generierte, lineare DNA-Fragmente sind.

19. Verfahren nach einem beliebigen der Ansprüche 1 bis 18, wobei die Ausgangselternmatrizen in geeignete Vektoren, wie einem Plasmid, kloniert werden.

20. Verfahren nach einem beliebigen der Ansprüche 1 bis 19, wobei die Ausgangselternmatrizen ein Polynukleotid repräsentieren, das ein Enzym kodiert, wie eine Carbonylhydrolase, Carbohydrase oder Esterase, wie eine Protease, Lipase, Amylase, Cellulase, Oxidase oder Oxidoreduktase.

21. Verfahren nach einem beliebigen der Ansprüche 1 bis 19, wobei die Ausgangselternmatrizen ein Polynukleotid repräsentieren, das ein Polypeptid mit biologischer Aktivität kodiert, wie Insulin, ACTH, Glucagon, Somatostatin, Somatotropin, Thymosin, Parathyroidhormon, Hypophysenhormone, Somatomedin, Erythropoetin, luteinisierendes Hormon, Choriongonadotropin, Freisetzungsfaktoren des Hypothalamus, antidiuretische Hormone, Thyrioidea stimulierendes Hormon, Relaxin, Interferon, Thrombopoetin (TPO) und Prolaktin.

22. Verfahren nach einem beliebigen der Ansprüche 1 bis 19, wobei die Ausgangselternmatrizen ein Polynukleotid repräsentieren, enthaltend eine biologische Funktion, wie Transkriptionsinitiierung (Promoter), oder Termination, translationale Initiierung, Expression von Genen betreffende Operatorstellen.

23. Verfahren nach einem beliebigen der Ansprüche 1 bis 22, wobei besagte Anzahl an Zyklen weniger als 500, 200 oder weniger als 100 Zyklen ist.

## Revendications

1. Procédé de construction d'une banque de polynucléotides homologues recombinés à partir d'un certain nombre de différentes matrices d'ADN parentales de départ simple ou double brin et d'amorces par des décalages de matrices induits au cours d'une synthèse de polynucléotides *in vitro* en utilisant une polymérase, dans lequel
A) des amorces ou des polynucléotides étendus sont synthétisés par
a) dénaturation d'une matrice d'ADN parentale double brin pour produire des matrices simple brin,
b) hybridation desdites amorces aux matrices d'ADN simple brin,
c) extension desdites amorces par initiation de la synthèse par utilisation de ladite polymérase,
d) arrêt de la synthèse par élévation de la température ou par ajout de DMSO, et
e) dénaturation du double brin pour séparer les amorces étendues des matrices ;
B) un décalage de matrice est induit par
a) isolement des amorces étendues simple brin nouvellement synthétisées des matrices, et répétition des étapes (A)b) à (A)e) en utilisant lesdites amorces étendues produites dans (A) comme à la fois amorces et matrices, ou
b) répétitions des étapes (A)b) à (A)e) ;
C) le procédé ci-dessus est terminé après un nombre approprié de cycles des étapes du procédé (A) et (B)a), (A) et (B)b), ou des combinaisons de celles-ci ; et
D) facultativement, les polynucléotides produits sont amplifiés dans une réaction de PCR classique avec des amorces spécifiques pour amplifier de manière sélective des polynucléotides homologues d'intérêt.

2. Procédé selon la revendication 1, dans lequel ladite polymérase est une ADN polymérase thermostable.

3. Procédé selon la revendication 1, dans lequel ladite polymérase est une ADN polymérase, telle que la T4 polymérase, la T7 polymérase, l'ADN polymérase I ou le fragment de Klenow de l'ADN polymérase I de E. coli, et ladite polymérase est ajoutée au mélange réactionnel après chaque cycle.

4. Procédé selon la revendication 1, dans lequel ladite polymérase est une ARN polymérase.

5. Procédé selon l'une quelconque des revendications 1 à 4, dans lequel ladite polymérase est une polymérase encline à l'erreur, telle qu'une transcriptase inverse comme la transcriptase inverse du VIH.

6. Procédé selon l'une quelconque des revendications 1 à 5, dans lequel lesdites matrices parentales de départ simple ou double brin sont différentes en ce qu'elles contiennent différentes mutations ponctuelles dans le même polynucléotide natif, ou sont des polynucléotides homologues isolés de la nature.

7. Procédé selon la revendication 6, dans lequel lesdites matrices parentales de départ simple ou double brin présentent une identité au niveau de l'ADN, par exemple, de plus de 95 %, 90 %, 85 %, 80 %, 75 %, 70 %, 65 %, 60 %, 55 %, ou même plus de 50 % d'identité.

8. Procédé selon l'une quelconque des revendications 1 à 7, dans lequel la température est élevée à une valeur entre 90°C et 99°C, de préférence 94°C.

9. Procédé selon l'une quelconque des revendications 1 à 8, dans lequel lesdites amorces comprennent une population d'amorces complètement aléatoires.

10. Procédé selon l'une quelconque des revendications 1 à 8, dans lequel lesdites amorces comprennent une population d'amorces semi-aléatoires.

11. Procédé selon l'une quelconque des revendications 1 à 8, dans lequel lesdites amorces comprennent une population d'amorces spécifiques.

12. Procédé selon l'une quelconque des revendications 1 à 11, dans lequel lesdites amorces sont marquées, de préférence avec de la biotine ou de la digoxigénine.

13. Procédé selon l'une quelconque des revendications 9, 10 ou 12, dans lequel lesdites amorces aléatoires ou semi-aléatoires ont une longueur de 6 à 500 pb, de préférence de 15 à 300 pb, et mieux de 25 à 150 pb.

14. Procédé selon l'une quelconque des revendications 11 ou 12, dans lequel lesdites amorces sont longues de 6 à 75 pb.

15. Procédé selon l'une quelconque des revendications 1 à 14, dans lequel lesdites matrices d'ADN parentales de départ sont homologues à au moins 50 %, 60 %, 70 %, 80 %, 90 % ou 95 %.

16. Procédé selon la revendication 15, dans lequel lesdites matrices d'ADN parentales de départ proviennent d'organismes de type sauvage de genres différents.

17. Procédé selon l'une quelconque des revendications 15 ou 16, dans lequel lesdites matrices parentales de départ ont une longueur d'environ 50 pb à 20 kb, de préférence environ 100 pb à 10 kb, de manière davantage préférée environ 200 pb à 7 kb, de manière spéciale environ 400 pb à 2 kb.

18. Procédé selon l'une quelconque des revendications 1 à 17, dans lequel lesdites matrices parentales de départ sont des fragments d'ADN linéaires générés par une réaction de PCR.

19. Procédé selon l'une quelconque des revendications 1 à 18, dans lequel lesdites matrices parentales de départ sont clonées dans des vecteurs appropriés, tels qu'un plasmide.

20. Procédé selon l'une quelconque des revendications 1 à 19, dans lequel lesdites matrices parentales de départ représentent un polynucléotide codant pour une enzyme, telle qu'une carbonyl-hydrolase, carbohydrase ou estérase, telle qu'une protéase, lipase, amylase, cellulase, oxydase ou oxydoréductase.

21. Procédé selon l'une quelconque des revendications 1 à 19, dans lequel lesdites matrices parentales de départ représentent un polynucléotide codant pour un polypeptide possédant une activité biologique, tel que l'insuline, l'ACTH, le glucagon, la somatostatine, la somatotropine, la thymosine, la parathormone, des hormones hypophysaires, la somatomédine, l'érythropoïétine, l'hormone lutéinisante, la gonadotropine chorionique, des facteurs de libération hypothalamiques, des hormones antidiurétiques, l'hormone de stimulation de la thyroïde, la relaxine, l'interféron, la thrombopoïétine (TPO) et la prolactine.

22. Procédé selon l'une quelconque des revendications 1 à 19, dans lequel lesdites matrices parentales de départ représentent un polynucléotide contenant une fonction biologique telle qu'une initiation (promoteur) ou une terminaison de la transcription, une initiation de la traduction, des sites opérateurs liés à l'expression de gènes.

23. Procédé selon l'une quelconque des revendications 1 à 22, dans lequel ledit nombre de cycles est inférieur à 500, 200 ou inférieur à 100 cycles.
